# EUROPEAN PATENT APPLICATION

(11) **EP 4 180 384 A1**
(43) Date of publication of application: **17.05.2023**
(21) Application number: 21837398.3
(22) Date of filing: 13.04.2021
(51) Int. Cl.: B81C 3/00, B01J 19/00, B81B 1/00, B81C 1/00, C12M 1/34, G01N 37/00

(54) **MICRO-CHANNEL DEVICE MANUFACTURING METHOD, AND MICRO-CHANNEL DEVICE**

(30) Priority: 07.07.2020 JP 2020116944
(71) Applicant: NOK Corporation, Tokyo 105-8585 (JP)
(72) Inventor: KOMORI, Takayuki, Fujisawa-shi, Kanagawa 2510042 (JP)
(74) Representative: Global IP Europe Patentanwaltskanzlei
(86) International application number: PCT/JP2021/015330
(87) International publication number: WO 2022/009493

(57) **Abstract**

There is provided a method of manufacturing a micro flow path device capable of easily manufacturing a micro flow path device having a pair of opposite electrodes on flow path sidewall surfaces that define the flow path. The method of manufacturing a micro flow path device comprising the steps of: arranging a cover film 6 on a surface of a base film 1 on which an electrode pattern 5 made of a metallic thin film 3 has been formed and obtaining a flow path forming laminate 10; punching the obtained flow path forming laminate 10 along a shape of a flow path 12a so as to cut a part of the electrode pattern 5 and forming a punched portion 12 in which at least a pair of opposite electrodes 16a and 16b are exposed to a part of the punched cut surface; and disposing a first planar member 18 defining a bottom surface of the flow path 12a on a back surface side of the flow path forming laminate 10 on which the punched portion 12 has been formed and disposing a second planar member 19 defining a top surface of the flow path 12a on a front surface side of the flow path forming laminate 10.

## Description

### Technical Field

The present invention relates to a manufacturing method of a micro flow path device capable of easily manufacturing a micro flow path device having a pair of opposite electrodes on flow path sidewall surfaces that define the flow path through which fluids flow, and a micro flow path device.

### Description of the Related Art

At present, the bulk analysis in which a population of cells is collectively analyzed is used for the analysis of the cells. In this analysis, it is difficult to analyze only a specific cell because the data is acquired by the average value of the cell population. Therefore, research and development of a cell sorter technique for removing only an objective cell from a sample have been actively carried out.

For example, there are a method of attaching magnetic beads adhering only to an objective cell and magnetically attracting, a method of obtaining a specific cell by controlling flow in a chip, a method of electrically attracting cells, and the like (see, for example, Non-Patent Document 1).

Among the above-mentioned methods, the method of attaching magnetic beads which uses antibodies derived from epithelia in cells such as circulating tumor cells (CTC) is known that the properties of the cells change, and has a problem that the omission may occur.

In addition, the method of obtaining a specific cell by controlling a flow of a sample requires a minute space, and has a problem such as blood clogging. As a method using an electrical method, a method using a conductive thin film such as a transparent electrode (also referred to as an "ITO electrode") is used, but since the conductive thin film is a secondary electrode, control of an electric field is limited. Therefore, in order to realize three-dimensional electrodes, research has been actively carried out in recent years (for example, see Non-Patent Document 2).

As a manufacturing method of a micro flow path devices, for example, a method of laminating constituent members such as bases, flow paths, covers, and the like has been proposed (see, for example, Patent Document 1). In such a manufacturing method, when an electrode is arranged on a flow path through which fluids flow, it is conceivable to arrange the electrode on a top surface or a bottom surface of the flow path, arrange the electrode so as to cover the flow path surface with polymer or plating, or separately laminate the conductive electrode parts.

### Citation List

### Patent Document

[Patent Document 1] JP-A-2010-014407

### Non-Patent Document

[Non-Patent Document 1] Masahito Hayashi, et al., Present state and prospects of measurement technologies for analysis of circulating tumor cell, Cytometry Research, 2011, Vol. 21, No. 2, p. 1-6
[Non-Patent Document 2] Srinivasu Valagerahally Puttaswamy et al., Simple and low cost integration of highly conductive three-dimensional electrodes in microfluidic devices, Biomedical Microdevices,2015,17:4

### SUMMARY OF THE INVENTION

### Problem to be solved by the Invention

As shown in Non-Patent Document 2, a conventional cell sorter having a three-dimensional electrode structure is manufactured by forming a groove structure using soft lithography or the like in a portion having a three-dimensional electrode structure, and pouring a conductive slurry with a syringe or the like and solidifying. Such a method may have problems such as leakage of the conductive slurry to a portion not intended or insufficient filling, at the time of manufacturing. Furthermore, there is also a problem that a conductive structure of µm scale which is smaller than the conductive filler cannot be produced.

In view of the above-mentioned problems, the present invention provides a manufacturing method of a micro flow path device capable of easily manufacturing a micro flow path device having a pair of opposite electrodes on flow path sidewall surfaces that define the flow path through which fluids flow. The present invention also provides a micro flow path device having a pair of opposite electrodes on flow path sidewall surfaces that define the flow path through which fluids flow.

### Means for Solving the Problems

In order to solve the above problems, the present invention provides a manufacturing method of a micro flow path device and a micro flow path device described below.
[1] A method of manufacturing a micro flow path device provided with a flow path through which fluids flow comprising the steps of:
   forming an electrode pattern made of a metal thin film on a surface of a base film;
   arranging a cover film on the surface of the base film on which the electrode pattern is formed, and obtaining a flow path forming laminate that defines a side surface portion in the flow path of the micro flow path device;
   punching the obtained flow path forming laminate along the shape of the flow path so as to penetrate from the front surface to the back surface thereof and to cut a part of the electrode pattern, and forming a punched portion defining both side surfaces of the flow path in which at least a pair of opposite electrodes are exposed to a part of the punched cut surface,
   disposing a first planar member defining a bottom surface of the flow path on the back surface side of the flow path forming laminate on which the punched portion is formed and disposing a second planar member defining a top surface of the flow path on the front surface side of the flow path forming laminate on which the punched portion is formed.
[2] The method of manufacturing a micro flow path device according to [1], wherein the second planar member has a liquid inlet and a liquid outlet communicating with the inside and the outside at one end side and the other end side of the punched portion of the flow path forming laminate.
[3] The method of manufacturing a micro flow path device according to [1] or [2], wherein the step of forming the electrode pattern is performed by photoetching.
[4] The method of manufacturing a micro flow path device according to any one of [1] to [3], wherein a surface of the electrode pattern is nickel-gold-plated.
[5] The method of manufacturing a micro flow path device according to any one of [1] to [4], wherein the base film on which the electrode pattern is formed and the cover film are adhered to each other by an adhesive layer made of an adhesive for flexible printed circuits.
[6] A micro flow path device provided with a flow path through which fluids flow, wherein the micro flow path device has a pair of opposite electrodes on a part of flow path sidewall surfaces that define both side surfaces of the flow path.

### Effect of the Invention

In the manufacturing method of a micro flow path device of the present invention, a micro flow path device having a pair of opposite electrodes on flow path sidewall surfaces that define the flow path can be easily manufactured. That is, in the manufacturing method of a micro flow path device of the present invention, an electrode pattern made of a metallic thin film is formed on the surface of a base film, a cover film is disposed on the surface of the base film to obtain a flow path forming laminate, and the obtained flow path forming laminate is punched along the shape of a flow path, thereby manufacturing a flow path of the micro flow path device. Therefore, a part of the electrode pattern is exposed to a part of the cut surface of the punched portion to be the flow path, and a pair of opposite electrodes can be provided on the flow path sidewall surfaces that define both sides of the flow path. Then, the micro flow path device having the electrodes capable of generating a three-dimensional electric field can be manufactured very easily by sealing a top surface and a bottom surface of the flow path forming laminate in which the punched portion is formed. In particular, in the above-described manufacturing method, since the flow path is formed by punching the electrode pattern formed on the base film in advance, the formation of the electrodes to a portion not intended is suppressed, and the problems in the conventional manufacturing method such as leakage of the conductive slurry to a portion not intended or insufficient filling can be solved. Furthermore, since the method can form an electrode pattern using, for example, an etching technique of copper foil, without using a conductive slurry that requires a conductive filler, it is possible to produce a three-dimensional conductive structure of µm scale.

In the micro flow path device of the present invention, a three-dimensional electric field can be generated in the flow path by a pair of electrodes provided on the flow path sidewall surfaces that define the flow path. Therefore, it is possible to capture particles in the fluid by an electrical method, and to satisfactorily confirm how the particles flow along the fluid.

### BRIEF DESCRIPTION OF THE DRAWINGS

FIG. 1 is a schematic plan view showing one step of the manufacturing method of the micro flow path device of the first embodiment.
FIG. 2 is a cross-sectional view showing a cross-section taken along the line A1-A1' of FIG. 1.
FIG. 3 is a schematic plan view showing one step of the manufacturing method of the micro flow path device of the first embodiment.
FIG. 4 is a cross-sectional view showing a cross-section taken along the line A2-A2' of FIG. 3.
FIG. 5 is a schematic plan view showing one step of the manufacturing method of the micro flow path device of the first embodiment.
FIG. 6 is a cross-sectional view showing a cross-section taken along the line A3-A3' of FIG. 5.
FIG. 7 is a schematic plan view showing one step of the manufacturing method of the micro flow path device of the first embodiment.
FIG. 8 is a cross-sectional view showing a cross-section taken along the line A4-A4' of FIG. 7.
FIG. 9 is a cross-sectional view showing a cross-section taken along the line B4-B4' of FIG. 7.
FIG. 10 is a schematic plan view showing one step of the manufacturing method of the micro flow path device of the first embodiment.
FIG. 11 is a cross-sectional view showing a cross-section taken along the line A5-A5' of FIG. 10.
FIG. 12 is a cross-sectional view showing a cross-section taken along the line B5-B5' of FIG. 10.
FIG. 13 is a schematic plan view showing one step of the manufacturing method of the micro flow path devices of the first embodiment.
FIG. 14 is a cross-sectional view showing a cross-section taken along the line A6-A6' of FIG. 13.
FIG. 15 is a cross-sectional view showing a cross-section taken along the line B6-B6' of FIG. 13.
FIG. 16 is a photomicrograph taken when a voltage is applied to the cell suspension being fed, in the micro flow path device of Example 1.

### Mode for carrying out the invention

Embodiments of the present invention will be described below with reference to the drawings. It should be understood that the present invention is not limited to the following embodiments, and that appropriate design changes, improvements, and the like can be added based on ordinary knowledge of a person skilled in the art without departing from the spirit of the present invention.

### (1) Manufacturing method of micro flow path device:

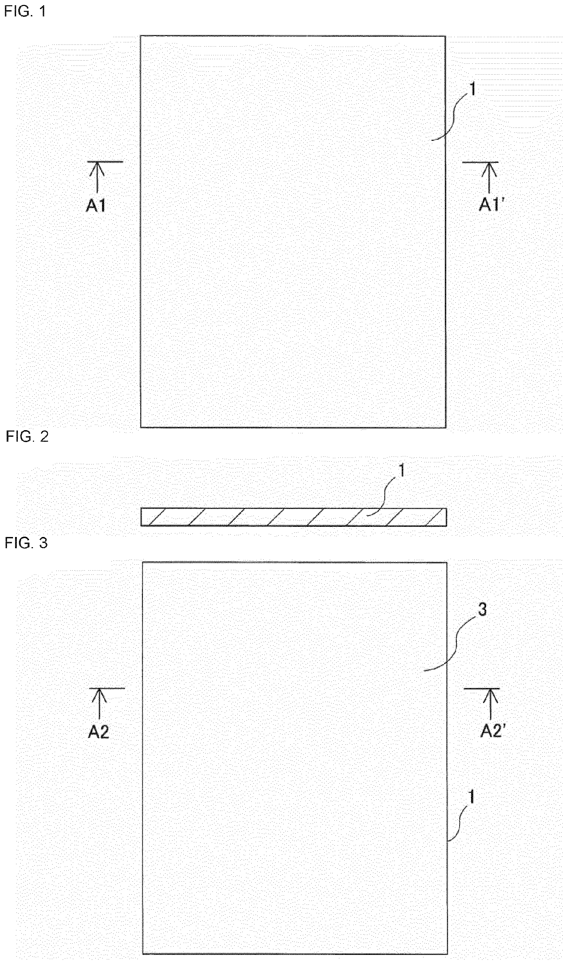

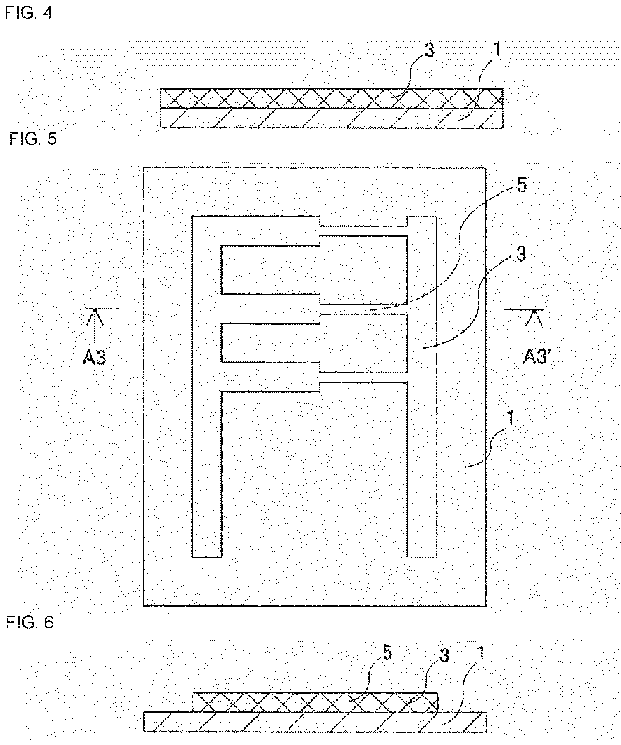

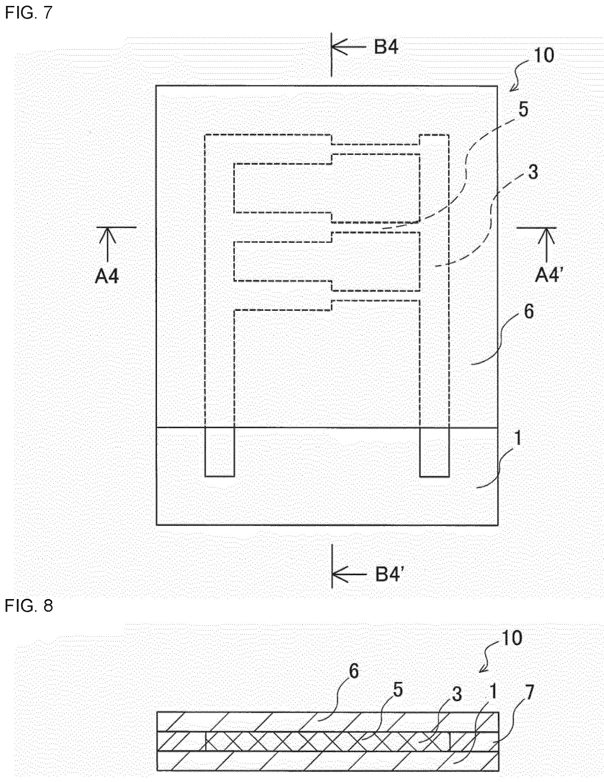

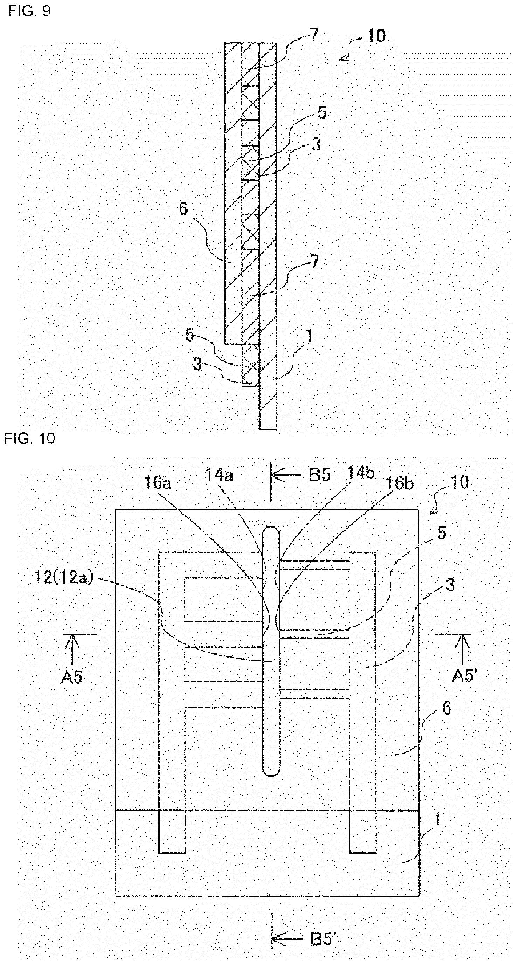

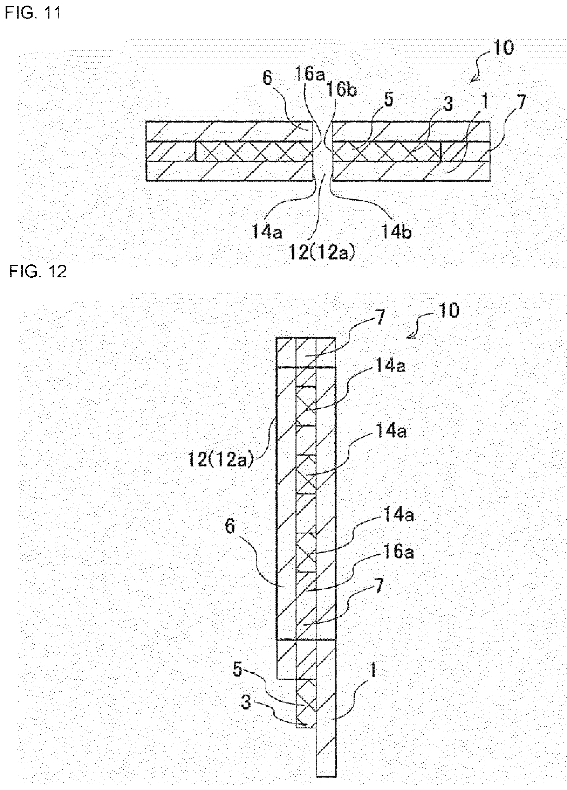

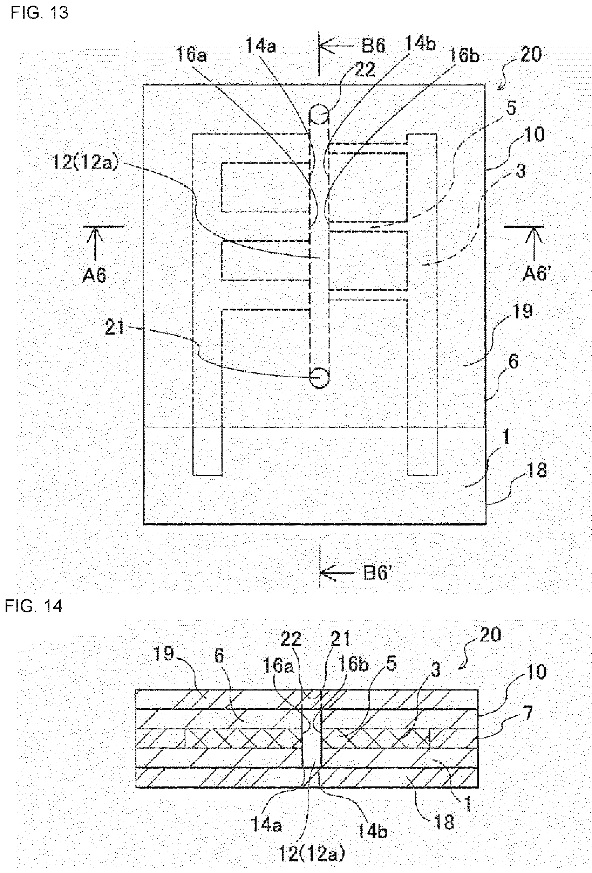

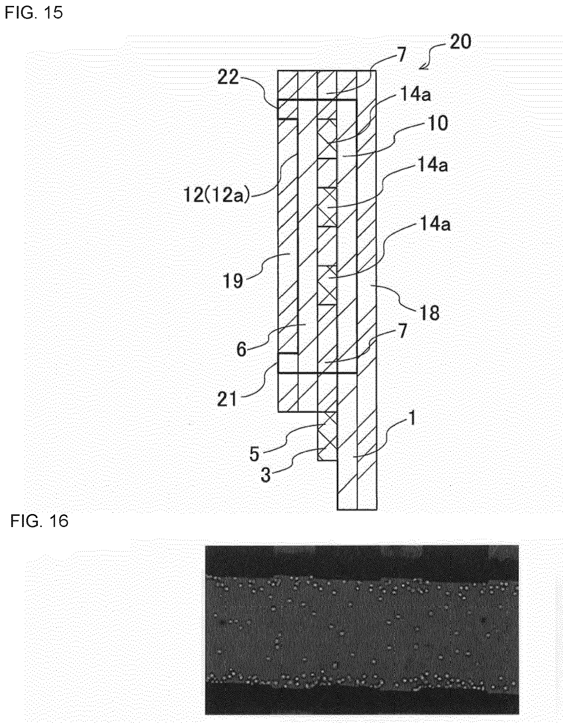

A manufacturing method of a micro flow path device of the present embodiment is a manufacturing method including the steps shown in FIGs. 1 to 15. Here, FIGs. 1, 3, 5, 7, 10, and 13 are plan views schematically showing each step of the manufacturing method of the micro flow path device of the first embodiment. FIG. 2 is a cross-sectional view showing a cross-section taken along the line A1-A1' of FIG. 1. FIG. 4 is a cross-sectional view showing a cross-section taken along the line A2-A2' of FIG. 3. FIG. 6 is a cross-sectional view showing a cross-section taken along the line A3-A3' of FIG. 5. FIG. 8 is a cross-sectional view showing a cross-section taken along the line A4-A4' of FIG. 7, and FIG. 9 is a cross-sectional view showing a cross-section taken along the line B4-B4' of FIG. 7. FIG. 11 is a cross-sectional view showing a cross-section taken along the line A5-A5' of FIG. 10, and FIG. 12 is a cross-sectional view showing a cross-section taken along the line B5-B5' of FIG. 10. FIG. 14 is a cross-sectional view showing a cross-section taken along the line A6-A6' of FIG. 13, and FIG. 15 is a cross-sectional view showing a cross-section taken along the line B6-B6' of FIG. 13. Hereinafter, the manufacturing method of a micro flow path device of the present embodiment may simply be referred to as the manufacturing method of the present embodiment.

The manufacturing method of the present embodiment is a method of manufacturing a micro flow path device 20 provided with a flow path 12a through which fluids flow, as shown in FIGs. 13 to 15, and in particular, a method of manufacturing a micro flow path device 20 having a pair of opposite electrodes 16a and 16b on a part of the flow path sidewall surfaces 14a and 14b that define both side surfaces of the flow path 12a. The micro flow path device 20 can generate a three-dimensional electric field in the flow path 12a by a pair of electrodes 16a and 16b provided on the flow path sidewall surfaces 14a and 14b. Hereinafter, a structure having electrodes 16a and 16b provided on the flow path sidewall surfaces 14a and 14b that define both side surfaces of the flow path 12a and capable of generating a three-dimensional electric field in the flow path 12a, as shown in FIGs. 13 to 15, may be referred to as a "three-dimensional electrode structure" and a "three-dimensional conductive structure".

In particular, the manufacturing method of the present embodiment is a method comprising: a step A of forming an electrode pattern made of a metal thin film on a surface of a base film; a step B of arranging a cover film on the surface of the base film on which the electrode pattern is formed, and obtaining a flow path forming laminate for defining a side surface portion in the flow path of the micro flow path device; a step C of punching the obtained flow path forming laminate along the shape of the flow path so as to penetrate from the front surface to the back surface thereof and to cut a part of the electrode pattern, and forming a punched portion defining both side surfaces of the flow path in which at least a pair of opposite electrodes are exposed to a part of the punched cut surface; and a step D of disposing a first planar member defining a bottom surface of the flow path on the back surface side of the flow path forming laminate on which the punched portion is formed, and disposing a second planar member defining a top surface of the flow path on the surface side of the flow path forming laminate on which the punched portion is formed. According to the manufacturing method of the present embodiment, a micro flow path devices having electrodes capable of generating a three-dimensional electric field can be manufactured extremely easily. In particular, since the flow path is formed by punching the electrode pattern formed on the base film in advance, the formation of the electrodes to a portion not intended is suppressed, and the problems in the conventional manufacturing method such as leakage of the conductive slurry to a portion not intended or insufficient filling can be solved. Furthermore, since the method can form an electrode pattern using, for example, an etching technique of copper foil, without using a conductive slurry that requires a conductive filler, a three-dimensional conductive structure of µm scale can be produced. Hereinafter, the manufacturing method of the present embodiment will be described in further detail for each step.

In a step A of forming an electrode pattern made of a metal thin film on a surface of a base film, first, a base film 1 as shown in FIGs. 1 and 2 is prepared. The base film 1 can be formed by, for example, a resin composition. In particular, as the base film 1 used in the manufacturing method of the present embodiment, a plastic film such as polyimide for flexible printed circuits (hereinafter, also referred to as "FPC") or polyester (PET) can be suitably used.

Next, in the step A, a metal thin film 3 is arranged on the surface of the base film 1 as shown in FIGs. 3 and 4. The metal thin film 3 is equivalent to the conductive foil in FPC. The material of the metal thin film 3 is not particularly limited. For example, copper/copper foil is generally used as the metal thin film 3. The metal thin film 3 can be adhered to the surface of the base film 1 by, for example, an epoxy resin type adhesive, an acrylic resin type adhesive, a prepreg, or the like. In FIGs. 3 and 4, the adhesive layer for adhering the base film 1 and the metal thin film 3 is omitted.

Next, in the step A, an electrode pattern 5 made of the metal thin film 3 is formed as shown in FIGs. 5 and 6. As a method of forming the electrode pattern 5, a photoetching technique in which a photoresist treatment (rust prevention treatment) is applied only to the necessary portion of the metal thin film 3 and the unnecessary portion is dissolved, eroded and etched by an etching reagent can be used. With such a configuration, the electrode pattern 5 having a desired shape can be easily formed. The formation of the electrode pattern 5 by etching can be performed, for example, in accordance with the wiring pattern preparation procedure in conventionally known FPC. For example, as an example of a method of forming the electrode pattern 5, the following method can be used. First, a photoresist is laminated on the surface of the base film 1 to which a copper foil as the metal thin film 3 is adhered, to dispose a dry film. Next, the electrode pattern 5 is transferred onto the dry film by irradiating ultraviolet rays. Next, a dry film of an unexposed part of ultraviolet rays is dissolved, and then a copper foil other than the electrode pattern 5 is chemically removed. Next, the dry film of an exposed part is dissolved. In this manner, the electrode pattern 5 made of the metal thin film 3 can be formed.

After forming the electrode pattern 5, gold plating may be applied on the portion where the electrode pattern 5 is exposed on the base film 1. Examples of the gold plating include nickel-gold plating. By applying gold plating to the electrode pattern 5, it is possible to adjust the thickness of the electrode pattern 5 in accordance with the height of the flow path 12a in the micro flow path device 20 as shown in FIGs. 13 to 15, and to provide a good biocompatibility.

In the electrode pattern 5, the exposed portions at the punched cut surface by the punching process in the step C described later become the electrodes 16a and 16b of the micro flow path device 20 as shown in FIGs. 13 to 15. The remaining portions of the electrode pattern 5 after the punching process become wirings (printed wirings) for electrically connecting to the electrodes 16a and 16b. Therefore, the shape of the electrode pattern 5 can be appropriately determined depending on the shape of the flow path 12a to be formed in the micro flow path device 20, the shape of the electrodes 16a and 16b provided in the flow path sidewall surfaces 14a and 14b of the flow path 12a, and the like.

Next, a step B of the manufacturing method of the present embodiment is arranging a cover film 6 on the surface of the base film 1 on which the electrode pattern 5 is formed as shown in FIGs 7 to 9, and obtaining a flow path forming laminate 10 for defining the side surface portions in the flow path 12a (see FIG. 13) of the micro flow path device 20 (see FIG. 13). The cover film 6 protects the electrode pattern 5 on the base film 1. As the cover film 6, a film similar to the cover lay film (surface protective film) in a conventionally known FPC can be used. When arranging the cover film 6, it is preferable to apply an adhesive to the surface of the base film 1 and bond the base film 1 and the cover film 6 together. An adhesive layer 7 made of the adhesive applied to the surface of the base film 1 is formed between the base film 1 and the cover film 6. The step B described above can also be performed in accordance with the manufacturing procedure of the conventionally known FPC.

Next, a step C of the manufacturing methods of the present embodiment is punching the obtained flow path forming laminate 10 along the shape of the flow path 12a so as to penetrate from the front surface to the back surface thereof and to cut a part of the electrode pattern 5, as shown in FIGs. 10 to 12, and forming a punched portion 12 defining both side surfaces (flow path sidewall surfaces 14a and 14b) of the flow path 12a in which at least a pair of opposite electrodes 16a and 16b are exposed to a part of the punched cut surface. The method of punching the flow path forming laminate 10 is not particularly limited, and a preferred example thereof is laser beam punching. The laser beam punching is easy to process, has a high degree of freedom in processing, can suppress the generation of burrs and flash, and can finish the punched cut surface finely.

Next, a step D of the manufacturing method of the present embodiment is disposing a first planar member 18 defining a bottom surface of the flow path 12a on the back surface side of the flow path forming laminate 10 on which the punched portion 12 is formed, and disposing a second planar member 19 defining a top surface of flow path 12a on the surface side of the flow path forming laminate 10, as shown in FIGs. 13 to 15. With this configuration, the micro flow path device 20 having a pair of opposite electrodes 16a and 16b on a part of the flow path sidewall surfaces 14a and 14b that define both side surfaces of the flow path 12a can be easily manufactured. The first planar member 18 and the second planar member 19 are disposed so as to cover the back surface side and the front surface side of the flow path forming laminate 10 to define the bottom and top surfaces of the punched portion 12 (in other words, the flow path 12a). For the first planar member 18 and the second planar member 19, a transparent polyester film or the like can be used. An adhesive (e.g., an acrylic adhesive) may be applied on one surface of the first planar member 18 and the second planar member 19.

The second planar member 19 may have a liquid inlet 21 and a liquid outlet 22 which communicate with the inside and the outside at one end side and the other end side of the punched portion 12 of the flow path forming laminate 10. The liquid inlet 21 and the liquid outlet 22 can be formed by punching on a predetermined portion of the second planar member 19. The liquid inlet 21 is an opening for introducing a liquid into the flow path 12a formed by the punched portion 12, and the liquid outlet 22 is an opening for discharging the liquid introduced into the flow path 12a from the liquid inlet 21 to the outside.

The micro flow path device 20 manufactured as described above is utilized in, for example, a field of bio, medical, healthcare, and the like. More specifically, it can be suitably used as a microanalysis chip, a microinspection chip, a microfluidic chip (µTAS; Micro Total Analysis Systems), for example.

### (2) Micro flow path device

Next, the micro flow path device of the present embodiment is a micro flow path device 20 provided with a flow path 12a through which fluids flow, as shown in FIGs. 13 to 15. In particular, the micro flow path device 20 is configured mainly to have a pair of opposite electrodes 16a and 16b on a part of the flow path sidewall surfaces 14a and 14b that defines both side surfaces of the flow path 12a. The micro flow path device 20 of the present embodiment can be manufactured by the manufacturing method of the present embodiment described above. That is, the flow path 12a of the micro flow path device 20 is formed by punching a part of the flow path forming laminate 10 as a structure in which the electrode pattern 5 made of the metallic thin film 3 is sandwiched between the base film 1 and the resin film such as the cover film 6. Therefore, a part of the electrode pattern 5 is exposed to a part of the cut surface of the punched portion 12 to be the flow path 12a, and a pair of opposite electrodes 16a and 16b are provided on the flow path sidewall surfaces 14a and 14b that define both side surfaces of the flow path 12a.

In the micro flow path device 20 of the present embodiment, a three-dimensional electric field can be generated in the flow path 12a by a pair of electrodes 16a and 16b provided on the flow path sidewall surfaces 14a and 14b that define the flow path 12a. Therefore, it is possible to capture particles in the fluid by an electrical method, and to confirm well how the particles flow along the fluid. Further, since a pair of electrodes 16a and 16b are provided on the flow path sidewall surfaces 14a and 14b that define the flow path 12a, the electrodes 16a and 16b can be prevented from contacting with upper and lower (i.e., top and bottom of the) wall surfaces that define the flow path 12a. In particular, when manufacturing by the manufacturing method described above, it is possible to easily realize a configuration in which the electrodes 16a and 16b are not in contact with the upper and lower wall surfaces of the flow path 12a, respectively. In the micro flow path device 20 thus configured, there is no interference by the electrodes 16a and 16b when observing the fluid with a microscope, and the fluid observation can be performed extremely well, as compared with one in which the electrodes 16a and 16b are provided on the upper and lower wall surfaces that define the flow path 12a.

### Examples

Hereinafter, the present invention will be described more specifically by way of Examples, but the present invention is not limited by these Examples in any way.

### (Example 1; Manufacturing micro flow path device)

First, a polyimide film was prepared as a base film, and a copper foil was adhered to one side of the prepared polyimide film.

Next, a photoresist was laminated on the copper foil adhered to the polyimide film to dispose a dry film. Then, the dry film was irradiated with ultraviolet rays to transfer a desired electrode pattern.

Next, a dry film of an unexposed part of ultraviolet rays was dissolved, and then, a copper foil other than the electrode pattern was chemically removed. Thereafter, the dry film of an exposed part was dissolved. In this manner, an electrode pattern made of copper foil was formed on the polyimide film.

Next, nickel-gold plating was performed on the portion where the electrode pattern is exposed. The nickel-gold plating is used to adjust the thickness of the electrode pattern in accordance with the height of the flow path in the micro flow path device to be manufactured. Such nickel-gold plating can also provide a biocompatibility to the micro flow path device to be manufactured.

Next, an adhesive was applied to the surface of the base film and bonded the base film and the cover film together to obtain a flow path forming laminate for defining the side surface portion in the flow path of the micro flow path device. As the cover film, a polyimide film was used. The manufacturing procedure of the above-described steps was performed in accordance with the manufacturing procedure of the conventionally known FPC composed of: a resin layer; an adhesive layer; a conductive layer; an adhesive layer; and a resin layer, except that nickel-gold plating was performed on the portion where the electrode pattern is exposed.

Next, a portion to be a flow path of the flow path forming laminate was penetrated using laser beam to form a punched portion in which at least a pair of opposite electrodes were exposed to a part of the punched cut surface. A carbon dioxide laser was used as a laser for penetrating a portion to be a flow path.

Next, a first planar member defining the bottom surface of the flow path was disposed on the back surface side of the flow path forming laminate in which the punched portion was formed, and a second planar member defining the top surface of the flow path was disposed on the front surface side of the flow path forming laminate. As the first planar member and the second planar member, "Spacer Tape 9964 (trade name)" manufactured by 3M was used. The second planar member was provided with a liquid inlet and a liquid outlet communicating with the inside and the outside at one end side and the other end side of the flow path.

As described above, the micro flow path device with a three-dimensional electrode structure having a pair of opposite electrodes on the flow path sidewall surfaces that define both side surfaces of the flow path, was manufactured. The micro flow path device thus manufactured was used as a micro flow path device of Example 1.

### (Preparation of sample fluid)

As a sample fluid for evaluating the micro flow path device of Example 1, a cell suspension containing calcein-stained Hela cells was prepared.

### (Evaluation of micro flow path device)

The cell suspension prepared as described above was fed to the flow path of the micro flow path device of Example 1. At this time, a voltage of 3MHz, 10V was applied to the electrodes of the micro flow path device of Example 1. FIG. 16 is a photomicrograph taken when the voltage was applied to the cell suspension being fed, in the micro flow path device of Example 1. As shown in FIG. 16, it was confirmed that the cells were attracted to the electrodes when the voltage described above was applied to the electrodes, and that the cells flowed along the fluids flowing through the flow path. Therefore, in the micro flow path device of Example 1, it was possible to capture various particles such as cells contained in a fluid using an electrical method, by the electric field generated by the electrodes of a three-dimensional electrode structure, and to satisfactory confirm how the particles flow along the fluid.

### Industrial Applicability

The method of manufacturing a micro flow path device and the micro flow path device can be utilized in the fields of bio, medical, healthcare, and the like.

### Description of Reference Numerals

1: base film
3: metal thin film
5: electrode pattern
6: cover film
7: adhesive layer
10: flow path forming laminate
12: punched portion
12a: flow path
14a, 14b: flow path sidewall surface
16a, 16b: electrode
18: first planar member
19: second planar member
20: micro flow path device
21: liquid inlet
22: liquid outlet

## Claims

1. A method of manufacturing a micro flow path device provided with a flow path through which fluids flow comprising the steps of:
forming an electrode pattern made of a metal thin film on a surface of a base film;
arranging a cover film on the surface of the base film on which the electrode pattern is formed, and obtaining a flow path forming laminate that defines a side surface portion in the flow path of the micro flow path device;
punching the obtained flow path forming laminate along the shape of the flow path so as to penetrate from the front surface to the back surface thereof and to cut a part of the electrode pattern, and forming a punched portion defining both side surfaces of the flow path in which at least a pair of opposite electrodes are exposed to a part of the punched cut surface,
disposing a first planar member defining a bottom surface of the flow path on the back surface side of the flow path forming laminate on which the punched portion is formed and disposing a second planar member defining a top surface of the flow path on the front surface side of the flow path forming laminate on which the punched portion is formed.

2. The method of manufacturing a micro flow path device according to claim 1, wherein the second planar member has a liquid inlet and a liquid outlet communicating with the inside and the outside at one end side and the other end side of the punched portion of the flow path forming laminate.

3. The method of manufacturing a micro flow path device according to claim 1 or 2, wherein the step of forming the electrode pattern is performed by photoetching.

4. The method of manufacturing a micro flow path device according to any one of claims 1 to 3, wherein a surface of the electrode pattern is nickel-gold-plated.

5. The method of manufacturing a micro flow path device according to any one of claims 1 to 4, wherein the base film on which the electrode pattern is formed and the cover film are adhered to each other by an adhesive layer made of an adhesive for flexible printed circuits.

6. A micro flow path device provided with a flow path through which fluids flow, wherein the micro flow path device has a pair of opposite electrodes on a part of flow path sidewall surfaces that define both side surfaces of the flow path.
